(19) Europäisches Patentamt / European Patent Office / Office européen des brevets

(11) EP 2 682 122 A1

(12) EUROPEAN PATENT APPLICATION
published in accordance with Art. 153(4) EPC

(43) Date of publication:
**08.01.2014 Bulletin 2014/02**

(21) Application number: **12754587.9**

(22) Date of filing: **02.03.2012**

(51) Int Cl.:
***A61K 31/7076*** (2006.01) ***A61K 36/06*** (2006.01)
***A61P 5/10*** (2006.01) ***A61P 43/00*** (2006.01)

(86) International application number:
**PCT/JP2012/055361**

(87) International publication number:
**WO 2012/121140 (13.09.2012 Gazette 2012/37)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **04.03.2011 JP 2011047942**
**20.05.2011 JP 2011113206**

(71) Applicant: **Lion Corporation**
**Tokyo 130-8644 (JP)**

(72) Inventors:
- **GOKITA, Toshio**
**Tokyo 130-8644 (JP)**
- **KAMADA, Ikuko**
**Tokyo 130-8644 (JP)**
- **SUZUKI, Kiwamu**
**Tokyo 130-8644 (JP)**
- **KAMBAYASHI, Hiroaki**
**Tokyo 130-8644 (JP)**

(74) Representative: **HOFFMANN EITLE**
**Patent- und Rechtsanwälte**
**Arabellastrasse 4**
**81925 München (DE)**

# (54) GROWTH HORMONE SECRETION PROMOTER

(57) It is an object of the present invention to provide a growth hormone secretagogue that is excellent in effect of promoting secretion of a growth hormone and is orally available. The growth hormone secretagogue of the present invention contains one or more selected from the group consisting of S-adenosylmethionine and/or a salt thereof, a Siberian larch extract, an olive extract, and a stilbene-based compound as an active ingredient. S-adenosylmethionine and/or the salt thereof may be a form itself of chemically synthesized S-adenosylmethionine and/or the salt thereof, or may be a microbial cell or a culture containing S-adenosylmethionine and/or the salt thereof. The stilbene-based compound may be an extract extracted from a *Vitaceae* plant or a *Gnetaceae* plant. The olive extract may be an extract separated from a fruit of olive.

EP 2 682 122 A1

## Description

### TECHNICAL FIELD

The present invention relates to a growth hormone secretagogue, and more particularly relates to a growth hormone secretagogue containing S-adenosylmethionine as an active ingredient.

### BACKGROUND

A growth hormone (hereinafter sometimes abbreviated as GH) is secreted from pituitary and involved in metabolism such as bone metabolism, protein metabolism, carbohydrate metabolism, fat metabolism, and electrolyte metabolism. Dysfunction of growth hormone secretion leads to disorders in development and growth in childhood, and is a risk factor of diseases such as metabolic disorders and cardiovascular disorders in adulthood. Even in healthy persons, the growth hormone secretion is reduced with aging. It is known that the growth hormone secretion is relatively more dysfunctional in the elderly than young adults. It is also pointed out that the dysfunction of the growth hormone secretion leads to reduced quality of life in the elderly.

There are findings that the disorders in the development and growth are improved by administering a growth hormone itself to the dysfunction of the growth hormone secretion in the childhood. There are also findings that the risk factor for the disease such as the metabolic disorder is reduced by administering the growth hormone itself to the dysfunction of the growth hormone secretion in the adulthood. There are also findings that the quality of life is enhanced by administering the growth hormone itself to the dysfunction of the growth hormone secretion in the elderly. However, in a method of administering the growth hormone itself, it is necessary to strictly control administration conditions such as a dosage and control the method itself by a medical professional. The growth hormone is a peptide, and thus is necessary to be administered not orally but by injection. The administration by injection is invasive, which is problematic.

Substances having an action of promoting the secretion of the growth hormone in vivo are termed as growth hormone secretagogues. Instead of administering the growth hormone itself, a method of promoting the secretion of the growth hormone using an orally available growth hormone secretagogue has been proposed. This method is thought to be a safer measure for the dysfunction of the growth hormone secretion because its action is milder compared with an action in the administration of the growth hormone itself.

Prebiotics such as dietary fibers and probiotics such as spore bearing lactobacillus (Patent Literature 1: Japanese Patent Application Laid-open No. 2007-131541-A), peptides derived from soybean protein (Patent Literature 2: Japanese Patent Application Laid-open No. 2006-347946-A), and γ-aminobutyric acid obtained by lactobacillus fermentation (Patent Literature 3: Japanese Patent Application Laid-open No. 2004-269361-A) have been proposed as orally available growth hormone secretagogues.

However, effects of the conventional growth hormone secretagogues have not been satisfied.

### RELATED ART DOCUMENTS

### PATENT LITERATURE

Patent Literature 1: Japanese Patent Application Laid-open No. 2007-131541-A
Patent Literature 2: Japanese Patent Application Laid-open No. 2006-347946-A
Patent Literature 3: Japanese Patent Application Laid-open No. 2004-269361-A

### SUMMARY OF THE INVENTION

### PROBLEM TO BE SOLVED BY THE INVENTION

It is an object of the present invention to provide a growth hormone secretagogue that is excellent in effect of promoting secretion of a growth hormone and is orally available.

### MEANS FOR SOLVING PROBLEM

As a result of an extensive study for solving the above problem, the present inventors have found that S-adenosylmethionine effectively promotes the secretion of the growth hormone.

The present invention has been made based on the above finding, and provides the growth hormone secretagogue employing the following constitutions.

[1] A growth hormone secretagogue comprising one or more selected from the group consisting of S-adenosylmethionine and/or a salt thereof, a Siberian larch extract, an olive extract, and a stilbene-based compound.
[2] The growth hormone secretagogue according to [1] above, wherein S-adenosylmethionine and/or the salt thereof is chemically synthesized S-adenosylmethionine and/or salt thereof.
[3] The growth hormone secretagogue according to [1] above, wherein S-adenosylmethionine and/or the salt thereof is a microbial cell containing S-adenosylmethionine and/or the salt thereof, or a culture thereof.
[4] The growth hormone secretagogue according to [3] above, wherein the microbial cell is yeast.
[5] The growth hormone secretagogue according to any one of [1] to [4] above, wherein the stilbene-based compound is an extract extracted from a plant containing the stilbene-based compound.
[6] The growth hormone secretagogue according to [5] above, wherein the extract extracted from the plant containing the stilbene-based compound is an extract extracted from a Vitaceae plant containing the stilbene-based compound or an extract extracted from a *Gnetaceae* plant containing the stilbene-based compound.
[7] The growth hormone secretagogue according to any one of [1] to [6] above, wherein the olive extract is an extract separated from a fruit of olive.
[8] The growth hormone secretagogue according to any one of [1] to [7] above, wherein the olive extract is an olive extract comprising one or more polyphenols selected from the group consisting of hydroxytyrosol, tyrosol, and oleuropein.
[9] A food and drink product, a pharmaceutical product or a quasi-pharmaceutical product comprising one or more selected from the group consisting of S-adenosylmethionine and/or a salt thereof, a Siberian larch extract, an olive extract, and a stilbene-based compound.

The present invention also provides the following methods of promoting the secretion of the growth hormone.

[10] A method for promoting secretion of a growt hormone *in vivo* by administering one or more selected from the group consisting of S-adenosylmethionine and/or a salt thereof, a Siberian larch extract, an olive extract, and a stilbene-based compound to a living body.
[11] The method for promoting the secretion of the growth hormone *in vivo* according to [10] above, wherein S-adenosylmethionine and/or the salt thereof is chemically synthesized S-adenosylmethionine and/or salt thereof.
[12] The method for promoting the secretion of the growth hormone *in vivo* according to [10] above, wherein S-adenosylmethionine and/or the salt thereof is a microbial cell or a culture thereof containing S-adenosylmethionine and/or the salt thereof.
[13] The method for promoting the secretion of the growth hormone *in vivo* according to [12] above, wherein the microbial cell is yeast.
[14] The method for promoting the secretion of the growth hormone *in vivo* according to [10] above, wherein the stilbene-based compound is an extract extracted from a plant containing the stilbene-based compound.
[15] The method for promoting the secretion of the growth hormone *in vivo* according to [10] above, wherein the extract extracted from the plant containing the stilbene-based compound is an extract extracted from a *Vitaceae* plant containing the stilbene-based compound or an extract extracted from a *Gnetaceae* plant containing the stilbene-based compound.
[16] The method for promoting the secretion of the growth hormone *in vivo* according to [10] above, wherein the olive extract is an extract separated from a fruit of olive.
[17] The method for promoting the secretion of the growth hormone in vivo according to [10] above, wherein the olive extract is an olive extract comprising one or more polyphenols selected from the group consisting of hydroxytyrosol, tyrosol, and oleuropein.
[18] A method for promoting secretion of a growth hormone *in vivo* by administering a food and drink product, a pharmaceutical product or a quasi-pharmaceutical product comprising one or more selected from the group consisting of S-adenosylmethionine and/or a salt thereof, a Siberian larch extract, an olive extract, and a stilbene-based compound to a living body.

## EFFECT OF THE INVENTION

According to the present invention, it is possible to provide a growth hormone secretagogue that is orally available and excellent in effect of promoting the secretion of the growth hormone.

## EMBODIMENTS FOR CARRYING OUT THE INVENTION

In the present invention, an active ingredient of the growth hormone secretagogue is one or more selected from the group consisting of S-adenosylmethionine and/or a salt thereof, a stilbene-based compound, an olive extract, and a Siberian larch extract. In the present invention, one selected from the above group may be used alone, or two or more may be used in combination as the active ingredient of the growth hormone secretagogue.

(1) S-adenosylmethionine and/or salt thereof

It has been conventionally known that S-adenosylmethionine acts as a methyl group donor in a methylation reaction *in vivo* and plays important roles in promotion of phospholipid metabolism as well as in methylation such as methylation of proteins and methylation of nucleic acids. However, it has not been known that the secretion of the growth hormone *in vivo* is promoted by administering S-adenosylmethionine and/or the salt thereof to a living body.

S-adenosylmethionine takes a structure in which adenosine and methionine are bound via methylsulfonyl. An L-isomer, a D-isomer and a DL-isomer are present as optical isomers of methionine. In the present invention, methionine in the S-adenosylmethionine structure may be any of the above optical isomers, but is preferably the L-isomer in terms of physiological activity.

Examples of the salts of S-adenosylmethionine may include sulfate, p-toluenesulfonate, sulfonate/p-toluenesulfonate, methanesulfonate, trifluoromethanesulfonate, 1,4-butanedisulfonate, 1,5-pentanesulfonate, phosphate, chloride, and bromide. Any of these salts can be used as the active ingredient of the growth hormone secretagogue according to the present invention.

In the present invention, S-adenosylmethionine and/or the salt thereof may be S-adenosylmethionine and/or the salt thereof obtained by chemical synthesis or may be a microbial cell containing S-adenosylmethionine and/or the salt thereof

The microbial cell containing S-adenosylmethionine and/or the salt thereof may be a microbial cell originally containing S-adenosylmethionine and/or the salt thereof or a microbial cell originally containing no S-adenosylmethionine and/or the salt thereof or containing it in a trace amount but changed to contain S-adenosylmethionine and/or the salt thereof using a method of producing S-adenosylmethionine and/or the salt thereof in the microbial cell. Example of the method of producing S-adenosylmethionine and/or the salt thereof in the microbial cell may include a method of culturing the microbial cell to produce S-adenosylmethionine and/or the salt thereof in the microbial cell (in the cell). In such a method, any of a resulting culture, a culture medium, a microbial cell obtained by cultivation, a dried microbial cell obtained by drying the microbial cell, a purified microbial cell product obtained by purifying the culture or the culture medium, and a dried purified microbial cell product obtained by drying the purified microbial cell product may be used.

A type of the microbial cell is not particularly limited, but is preferably yeast. A type of the yeast is not particularly limited as long as the yeast produces S-adenosylmethionine and is orally available. Examples thereof may include the yeast belonging to genus *Saccharomyces,* and *Saccharomyces cerevisiae* is suitable.

In the present invention, one S-adenosylmethionine and/or the salt thereof obtained from a certain origin or a certain production method may be used alone, or two or more thereof obtained from a different origin or a different production method may be used in combination.

When the growth hormone secretagogue according to the present invention is used as a pharmaceutical product, S-adenosylmethionine and/or the salt thereof is preferably chemically synthesized S-adenosylmethionine and/or salt thereof or the microbial cell or the culture containing S-adenosylmethionine and/or the salt thereof. When the growth hormone secretagogue according to the present invention is used as a food product, S-adenosylmethionine and/or the salt thereof is preferably the aforementioned microbial cell or culture, and more preferably the yeast or a culture thereof.

When the growth hormone secretagogue of the present invention contains S-adenosylmethionine and/or the salt thereof as the active ingredient, a dosage of the growth hormone secretagogue is not particularly limited as long as the effect of the present invention is not impaired, and may be changed appropriately depending on factors such as an age and a condition of a subject to be administered. When the subject to be administered has no problem such as excessive secretion of the growth hormone, the dosage per day for obtaining an objective effect is generally 1 to 2000 mg, preferably 10 to 1600 mg, more preferably 10 to 600 mg and still more preferably 20 to 100 mg as the dosage of S-adenosylmethionine and/or the salt thereof per day.

The yeast containing S-adenosylmethionine and/or the salt thereof is preferably dried yeast. In the present invention, a commercially available yeast product containing S-adenosylmethionine and/or the salt thereof may be used. For example, "SAMe-rich dry yeast (trade name)" manufactured by Mitsubishi Gas Chemical Company Inc., "Super Esse (trade name)" manufactured by Italy Gnostic, and "Amy (trade name)" manufactured by Iwata Chemical Co., Ltd. are available as the commercially available products.

(2) Stilbene-based compound

The stilbene-based compound is a compound having a stilbene skeleton. The stilbene skeleton is represented by a formula (I).

[Chemical 1]

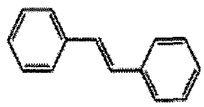

(I)

The stilbene skeleton may be substituted with a functional group. A site to be substituted with the functional group is not particularly limited, and is generally an aromatic ring. Examples of such a functional group may include a hydroxyl group, an alkyl group, a hydroxyalkyl group, an amino group, a sulfone group, and a phosphate group. Among them, the hydroxyl group and the hydroxyalkyl group are preferable.

The stilbene-based compound may be a monomer, a multimer, a monomeric glycoside or a multimeric glycoside as long as it has the stilbene skeleton. Among them, a monomer, a multimer or a glycoside thereof, a functional group of which is composed of a hydroxyl group or a hydroxyalkyl group is preferable. Examples of the monomer may include resveratrol, piceatannol, astringin, and pterostilbene. Examples of the monomeric glycoside may include piceid. Examples of the multimer may include $\delta$-viniferin, $\varepsilon$-viniferin, gnetin C, gnetin L, $\alpha$-viniferin, and ampelopsin A. Examples of the multimeric glycoside may include gnemonoside A, gnemonoside C, and gnemonoside D.

The above compounds are classified as resveratrols. Resveratrols are known to have an anti-ischemic heart disease effect (Lancet, 338, 1523-1526, 1992), an anti-cancer effect (Br. J. cancer, 86, 774-778, 2002), an effect of reducing development of dementia (Am. J. Epidemiol, 167, 648-691, 2008), a preventive effect on hypertension (Eur. J. Pharmacol., 667, 258-264, 2011), and an effect of prolonging a life (Nature, 444, 337-342, 2006). The stilbene-based compound is preferably one or more compounds selected from resveratrols.

In the present invention, the stilbene-based compound may be a stilbene-based compound obtained by chemical synthesis or a naturally occurring stilbene-based compound such as a stilbene-based compound derived from a microbial cell or a plant. Among them, the naturally occurring stilbene-based compound is preferable, and the stilbene-based compound derived from the plant is preferable.

The naturally occurring stilbene-based compound need not be a pure stilbene-based compound and may be a mixture with other compound(s). Examples of the mixture may include an extract extracted from a plant, a part or all of a plant body and a cultured microbial cell. Among them, the extract extracted from the plant is preferable.

When the extract extracted from the plant is used as the stilbene-based compound, it is possible to use an extract extracted from a plant containing the stilbene-based compound. Examples of the plant containing the stilbene-based compound may include *Vitaceae* plants, *Dipterocarpaceae* plants, *Leguminosae* plants, *Cyperaceae* plants, and *Gnetaceae* plants. Among them, the *Vitaceae* plants and the *Gnetaceae* plants are preferable.

The *Vitaceae* plant can contain the stilbene-based compound and preferably contains resveratrols. Examples of the *Vitaceae* plant may include species such as *Vitis vinifera, Vitis labrusca, Vitis californía, Vitis amurensis, Vitis coignetiae,* and *Vitis shiragai.* Examples of the *Vitaceae* plant may include breed varieties such as Cabernet sauvignon, Merlot, Pinot Noir, Gamay, Syrah, Nebbiolo, Sangiovese, Tempranillo, Zinfandel, Carmenere, Malbec, Muscat Bailey A, Koshu, Sauvignon Blanc, Semillon, Chardonnay, Muscadet, Riesling, Muscat, Viognier, Trebbiano, Pinot Grigio, Frumint, and Palomino.

The *Gnetaceae* plant is not particularly limited as long as it contains resveratrols. Examples of the *Gnetaceae* plant may include *Gnetum gnemon* (melinjo), *Gnetum brunonianum, Gnetum latifolium,* and *Gnetum tenuifolium.* Among them, *Gnetum gnemon* is preferable.

A site to be extracted in the plant is not particularly limited, and may be a whole plant body or a part thereof such as a leaf, a stem, a vine, a leave, a fruit and a seed. In the case of the *Vitaceae* plant, a stem, a vine, a leaf or a fruit (including fruit skins and seeds) are preferable. In the case of the *Gnetaceae* plant, a fruit, a seed, an endosperm in the fruit, a flower or a leaf is preferable, the fruit, the seed or the endosperm in the fruit is more preferable, and the endosperm in the fruit is still more preferable.

When a part of the plant body is used, a corresponding portion separated from the plant body may be used directly, or may be dried in the sun or by a machine for the use.

An extraction method is not particularly limited, and examples thereof may include a method of extracting using a solvent and a method of extracting using a supercritical extraction method by carbon dioxide and the like. Examples of an extraction solvent in the method of extracting using the solvent may include water, methanol, ethanol, n-propanol,

isopropanol, acetone, methyl ethyl ketone, methyl butyl ketone, ethyl acetate, ethylene glycol, propylene glycol, glycerin, acetic acid, and propionic acid. These extraction solvents may be used alone or in combination of any two or more as a mixture. The extraction solvent is preferably water, ethanol, or a mixed solution thereof (aqueous ethanol). When aqueous ethanol is used, an alcohol concentration is not limited, but is preferably 20 to 90% by volume and more preferably 40 to 80% by volume.

The extract extracted from the plant may be used, or the stilbene-based compound obtained by purifying the extract extracted from the plant may be used.

A grape extract and a melinjo extract are commercially available, and the commercially available products may be used in the present invention. Example of commercially available grape extracts may include "VINEATROL20M (trade name)" (manufactured by ACTICHEM Co.). Example of commercially available melinjo extracts may include "Melinjo Resveratrol-20 (trade name)" (manufactured by Luna YBF Corporation Inc.) .

When the growth hormone secretagogue of the present invention contains the stilbene-based compound as the active ingredient, the dosage of the growth hormone secretagogue is not particularly limited as long as the effect of the present invention is not impaired, and may be changed appropriately depending on factors such as the age and the condition of the subject to be administered. When the subject to be administered has no problem such as excessive secretion of the growth hormone, the dosage per day for obtaining the objective effect is generally 0.1 to 1000 mg, preferably 1 to 500 mg, more preferably 10 to 200 mg and still more preferably 20 to 120 mg as an ingestion of the stilbene-based compound per day.

(3) Olive extract

The olive extract has been conventionally known to have an anti-oxidative activity, preventive effects on heart diseases and cancers (Free Radical Biology & Medicine, 40, 608-616, 2006) and anti-inflammatory actions (J. Nutr., 1475-1479, 2005). However, the secretion of the growth hormone *in vivo* is promoted by administering the olive extract to a living body.

The olive extract is an extract separated from a whole or a part of plant body of olive *(Olea europaea L.).* The olive extract may be separated from a whole or a part of plant body of olive using the extraction solvent or separated by squeeze.

A site to be extracted from olive is not particularly limited, and may be a whole plant body or a part thereof such as a leaf, a flower, and a fruit, but a fruit is preferable.

For example, water, an organic solvent (e.g., one solvent or a mixed solvent of two or more selected from alcohol, acetone, and the like), or a mixed solution of water and the organic solvent may be used as the extraction solvent. Among them, water, the mixed solution of water and alcohol, and alcohol are preferable. Ethanol and methanol are preferable as alcohol. An extraction time period and temperature may be determined appropriately depending on the site to be extracted in olive and the type of the solvent. The olive extract may be a crude extract extracted from olive or one obtained by giving a treatment such as concentration, drying, and pulverization to the crude extract. Further, it is possible to use those obtained by removing impurities by a treatment by a partition method, and a purification treatment (e.g., absorption using an ion exchange resin and a column and subsequent elution with a solvent followed by concentration if necessary).

When the olive extract is separated by the squeeze, a target to be separated is preferably a fruit. A completely mature fruit of olive is more preferable. For example, the olive extract can be obtained by filtrating and/or concentrating a fruit juice obtained by squeezing the fruit. These filtrated and concentrated ones may be powderized by spray drying.

A form of the olive extract is not particularly limited and may be powder or paste.

Olive is generally rich in glyceride of fatty acids such as glyceride of oleic acid, glyceride of linoleic acid, and glyceride of palmitic acid, and lipid-soluble vitamins, and contains minerals such as potassium, phosphorus, and magnesium. Olive oil includes lipid-soluble ingredients such as unsaturated fatty acids, β-carotene, vitamin D, E, and squalene dihydroquercetin and polyphenol such as hydroxytyrosol, tyrosol, and oleuropein. In the present invention, it is preferable to remove pulp and oil, which are ingredient inherent to olive, from the olive extract by squeeze or concentration such as centrifugation.

In the present invention, the olive extract preferably contains polyphenol, and more preferably contains one or more polyphenols selected from the group consisting of hydroxytyrosol, tyrosol, and oleuropein. It is preferable that a content of polyphenol in the olive extract be high, and it is more preferable that the content of one or more polyphenols selected from the group consisting of hydroxytyrosol, tyrosol, and oleuropein be high.

The olive extract is commercially available, and the commercially available products may be used. Examples of the commercially available olive extract may include "Olivex (trade name)" (GROUPE GRAP'SUD, France).

When the growth hormone secretagogue of the present invention contains the olive extract as the active ingredient, the dosage of the growth hormone secretagogue is not particularly limited as long as the effect of the present invention is not impaired, and may be changed appropriately depending on factors such as the age and the condition

of the subject to be administered. When the subject to be administered has no problem such as excessive secretion of the growth hormone, the dosage per day for obtaining the objective effect is 0.1 to 1000 mg, preferably 1 to 500 mg, more preferably 10 to 200 mg and still more preferably 20 to 180 mg as the dosage of the olive extract per day.

(4) Siberian larch extract

A Siberian larch extract has been conventionally known to have an anti-oxidative activity and an inhibitory action on melanin synthesis. However, it has not been known that the secretion of the growth hormone *in vivo* is promoted by administering the Siberian larch extract to the living body.

The Siberian larch extract is an extract of Siberian larch *(Larix sibirica)* that is a Pinaceae needle-leaved tree distributed in Siberia and the Far East. The Siberian larch extract is composed mainly of dihydroquercetin and contains other ingredients such as dihydrokaempferol and naringenin in trace amounts.

The Siberian larch extract may be extracted from a whole plant body or a part of Siberian larch using an extraction solvent. A site to be extracted in Siberian larch is not particularly limited, and may be the whole plant body or a part thereof such as a leaf, a bark, a woody portion, a seed and a flower. The bark and the woody portion are preferable, and the woody portion is more preferable. For example, water, an organic solvent (e.g., one solvent or a mixed solvent of two or more selected from alcohol, acetone, and the like), or a mixed solution of water and the organic solvent may be used as the extraction solvent. Among them, water, the mixed solution of water and alcohol, and alcohol are preferable. Ethanol and methanol are preferable as alcohol. An extraction time period and temperature may be determined appropriately depending on the site to be extracted in Siberian larch and the type of the solvent. The Siberian larch extract may be a crude extract extracted from Siberian larch or one obtained by giving a treatment such as concentration, drying, pulverization, and squeeze to the crude extract. Further, it is possible to use those obtained by removing impurities by a treatment by a partition method, and a purification treatment (e.g., absorption using an ion exchange resin and a column and subsequent elution with the solvent followed by concentration if necessary). Meanwhile, a product obtained by finely pulverizing Siberian larch, wetting it with vapor followed by extracting with acetone to purify it, as described in Russia Patent No. 2091076, may be used. A form of the Siberian larch extract is not particularly limited and may be powder or paste.

The Siberian larch extract is commercially available and the commercially available products may be used in the present invention. Examples of the commercially available product of the Siberian larch extract may include "Jikuberuchin (trade name)" (Furabiru's, Irkutsk, Russia).

When the growth hormone secretagogue of the present invention contains the Siberian larch extract as the active ingredient, the dosage of the growth hormone secretagogue is not particularly limited as long as the effect of the present invention is not impaired, and may be changed appropriately depending on factors such as the age and the condition of the subject to be administered. When the subject to be administered has no problem such as excessive secretion of the growth hormone, the dosage per day for obtaining the objective effect is generally 3 to 1000 mg, preferably 15 to 500 mg, more preferably 20 to 200 mg and still more preferably 30 to 120 mg as the dosage of the Siberian larch extract per day.

In the present invention, one Siberian larch extract obtained under a particular extraction condition may be used alone, or two or more obtained under a different extraction condition may be used in combination.

The growth hormone secretagogue according to the present invention may consist only of one or more selected from the group consisting of S-adenosylmethionine and/or the salt thereof, the Siberian larch extract, the olive extract and the stilbene-based compound (i.e., one or more selected from the above may occupy 100% by mass of the entire secretagogue), or may comprise other ingredient(s). Examples of the other ingredient may include ingredients such as a storage stabilizer, which primarily ensure stability in storage and distribution, and all or a part of ingredients that compose an objective final product.

(Use of growth hormone secretagogue of the present invention for final product)

The growth hormone secretagogue of the present invention contains one or more selected from the group consisting of S-adenosylmethionine and/or the salt thereof, the stilbene-based compound, the olive extract and the Siberian larch extract having the effect of promoting the secretion of the growth hormone as the active ingredient. S-adenosylmethionine and/or the salt thereof is a substance originally contained in the living body. The stilbene-based compound is contained in the plants such as *Vitaceae* plant and *Gnetaceae* plants, the fruits of these plants are generally eaten, and their stems and vines are also eaten as cooked foods. Young leaves, flowers and fruits of the *Gnetaceae* plants are eaten as vegetables, and seeds of the *Gnetaceae* plants are usually eaten by crushing and drying them to obtain dry seeds and flying the dry seeds in oil in Southeast Asia such as Indonesia. Fruits of olive are generally eaten. The Siberian larch extract was previously eaten by native inhabitants in Eastern Russia, thus is safe when administered to the living body, and can effectively promote the secretion of the growth hormone. Therefore, any of these active

ingredients is safe when administered to the living body, and can effectively promote the secretion of the growth hormone.

The growth hormone secretagogue of the present invention may be used in unchanged form directly as a final product such as a food and drink product, a pharmaceutical product, a quasi-pharmaceutical product. The growth hormone secretagogue of the present invention may also be used as an additive for the food and drink product, the pharmaceutical product, and the quasi-pharmaceutical product. This can ameliorate or alleviate the dysfunction of the growth hormone secretion in the living body.

The growth hormone secretagogue of the present invention may comprise an ingredient other than the above active ingredients (pharmacologically acceptable base). One example of the ingredient other than the active ingredients may include the ingredients that primarily ensure the stability in the storage and the distribution such as storage stabilizer. In addition, the growth hormone secretagogue of the present invention may contain one or more (preferably one to three, and more preferably one) ingredients selected from various ingredients that compose the final product such as the food and drink product, the pharmaceutical product, the quasi-pharmaceutical product.

The ingredients other than the above active ingredients, which can be contained in the growth hormone secretagogue of the present invention are not particularly limited as long as the object of the present invention is not impaired. Examples thereof may include excipients, disintegrants, binding agents, lubricants, coating agents, coloring agents, color former, taste-masking agents, flavoring agents, antioxidants, preservatives, tasting agents, acidic-flavoring agents, sweeteners, nutrient suppliments, vitamin agents, leavening agents, thickeners, and surfactants. Among them, it is possible to select one or two or more that do not impair various properties required for formulation such as formulation stability and are suitable for a dosage form of the final product such as the pharmaceutical product, the quasi-pharmaceutical product, and the food and drink product. The other ingredient having the effect of promoting the growth hormone secretion may also be combined.

When the growth hormone secretagogue contains two or more active ingredients or the ingredient other than the active ingredients, it can also be rephrased as a growth hormone secretagogue composition.

(Administration form of growth hormone secretagogue of the present invention)

An administration form of the growth hormone secretagogue of the present invention is not particularly limited. Examples of the administration form may include oral administration such as intraoral administration and sublingual administration, and parenteral administration such as intravenous administration, intramuscular administration, subcutaneous administration, transdermal administration, transnasal administration, transpulmonary administration. Among them, the administration form that is less invasive is preferable, and the oral administration is more preferable.

A dosage form of the growth hormone secretagogue of the present invention is not particularly limited, and may be determined appropriately depending on any one of the food and drink product, the pharmaceutical product or the quasi-pharmaceutical product. In the case of the oral administration, examples of the dosage form may include liquid (liquid agent), syrup (syrup agent), tablet (tablet agent), capsule (capsule agent), powder (granule, fine particle), soft capsule (soft capsule agent), liquid (liquid agent), syrup (syrup agent), solid, semi-liquid, cream, and paste. Examples of the dosage form in the case of the parenteral administration may include dosage forms for the intravenous administration, the intramuscular administration, the subcutaneous administration, the transdermal administration, the transnasal administration, and the transpulmonary administration. Among them, the administration form that is no invasive for the living body is preferable, and the oral administration is preferable.

The administration form when the growth hormone secretagogue of the present invention is used as the food and drink product is generally the oral administration.

The growth hormone secretagogue of the present invention can promote the secretion of the growth hormone, and thus, is useful as the food and drink product, the pharmaceutical product and the quasi-pharmaceutical product for alleviating the dysfunction of the growth hormone secretion.

A subject who takes the growth hormone secretagogue of the present invention is not particularly limited. For example, the the growth hormone secretagogue of the present invention is suitable for subjects who worry about their growth and development disorder, subjects who have a sense of crisis for diseases such as metabolic disorders and cardiovascular disorders, and subjects who would like to enhance their quality of life. Even a subject who has no specific reason can routinely take it for the purpose of promoting the secretion of the growth hormone. An ingestion time period of the growth hormone secretagogue of the present invention is not particularly limited.

The growth hormone secretagogue of the present invention can be utilized as various food and drink products. Examples thereof may include beverages such as soft drinks, carbonated drinks, energy drinks, powdered drinks, fruit drinks, milk drinks, and jelly drinks, confectionaries such as cookies, cakes, chewing gums, candies, tablets, gummies, "manju" (steamed yeast buns with filling), "yokan" (adzuki bean jellies), puddings, jellies, ice creams, and sherbets, processed marine products such as "kamaboko" (boiled fish pastes), "chikuwa" (tubular rolls of boiled fish paste), and "hanpen" (puffy cakes made of ground fish), processed livestock products such as hamburgers, hams, sausages, wiener sausages, cheeses, butters, yogurt, fresh cream, cheeses, margarine, and fermented milk, soups such as powdered

soups and liquid soups, staple foods such as rice, noodles (dry noodles, uncooked noodles)), breads, cereal, and the like), and seasonings such as mayonnaise, shortening, dressings, sauces, "tare" (mop sauce), and soy sauce. The growth hormone secretagogue of the present invention can be utilized as the food and drink products such as healthy foods, functional foods, dietary supplements (supplements), foods for specified health use, foods for medical use, foods for the sick, baby foods, foods for nursing care, and foods for the elderly. Among them, it is used preferably as the healthy food and the functional food and more preferably as the healthy food.

The effect of promoting the secretion of the growth hormone in the present invention can be identified by administering a sample to a panelist before going to bed and detecting an increased amount of the growth hormone in urine in the following morning compared with an amount when the growth hormone secretagogue is not taken.

## EXAMPLES

The present invention will be described in more detail with reference to following Examples, but the present invention is not limited to these Examples.

Example 1 and Comparative Example 1

Tablets (80 mg of S-adenosylmethionine was contained in 9 tablets) in which yeast containing S-adenosylmethionine had been formulated were prepared as a sample of Example 1. A placebo tablet that had contained no yeast containing S-adenosylmethionine was prepared as a sample of Comparative Example 1. "Amy (trade name)" manufactured by Iwata Chemical Co., Ltd. was used as the yeast containing S-adenosylmethionine.

Each composition of the sample of Example 1 and the sample of Comparative Example 1 was as shown in following Table 1.

Table 1

| Ingredients (one dosage)* | Example 1 | Comparative Example 1 |
|---|---|---|
| Content of S-adenosylmethionine (mg) in yeast containing S-adenosylmethionine | 80.0 | 0.0 |
| Weight (mg) of yeast containing S-adenosylmethionine (dry yeast) | 1530.0 | 0.0 |
| Excipient (crystalline cellulose) (mg) | 270.0 | 1800.0 |
| *Amount in 9 tablets | | |

(Evaluation of effect of promoting growth hormone secretion)

Thirteen adult males and females as panelists ingested the sample of Example 1 one hour before going to bed, and an amount of growth hormone in the first urine (early morning first urine) upon wake-up in the following morning was measured. The same 13 panelists as in Example 1 ingested the sample of Comparative Example 1 one hour before going to bed, and an amount of the growth hormone in the first urine upon wake-up in the following morning was measured. The administration of each sample and the measurement were repeated three times per panelist. An average sleeping time of each panelist was 6.5 hours, a difference between the sleeping time of the panelist whose sleeping time was the longest and the sleeping time of the panelist whose sleeping time was the shortest was 2 hours, and the sleeping time of each panelist was not much different.

The effect of promoting the growth hormone secretion was evaluated by an increase of the amount of the growth hormone (GH) in the urine.

(Correlation between increase of GH amount in urine and increase of GH amount secreted *in vivo)*

It has been reported that the GH amount measured in the first urine in the early morning can be used as an alternative measurement of the GH amount measured by collecting the blood because the GH amount in the first urine in the early morning is well-correlated with the GH amount in blood for 3 hours after sleep onset in the night (Yasuko Itagane et al., "Clinical significance of measurement of growth hormone in urine", Pediatrics of Japan, vol. 80, No. 6, pages 629-636, 1989, Kanehara & Co., Ltd.).

(Measurement of GH amount in urine)

The GH amount in the urine was measured using a chemiluminescence enzyme immunoassay (CLEIA method), which was contracted out to a company for clinical laboratory testing (SRL Inc.). The CLEIA method is described in Adachi et al., Clinical Endocrinology, vol. 45, pages 223-235, 1997, Igakunosekaisha, Inc., and will be briefly explained below. First, GH in the urine is reacted with a murine anti-GH monoclonal antibody immobilized to wells in a microplate, further a biotinylated anti-GH antibody is reacted therewith, and then peroxidase-labeled streptoavidin is added thereto. Subsequently, when hydrogen peroxide and luminol are added thereto, luminescence with an intensity depending on the GH amount is generated. Thus, the intensity of the luminescence is measured, and the GH amount in the urine can be quantified by a standard curve. In the evaluation in present Examples, a creatinine concentration was also measured, and the GH amount corrected by the creatinine concentration was also calculated.

A mean value (mean value of urinary GH amounts after ingestion of S-adenosylmethionine) of measured values (three times) of the urinary GH amount (pg/mg·Cr) was calculated for each panelist. In Comparative Example 1, a mean value (mean value of urinary GH amounts after ingestion of placebo) was also similarly calculated. A change rate (%) of the urinary GH amounts was calculated for each panelist using a following formula (1).

Formula (1)

Change rate (%) of urinary GH amounts = (mean value of urinary GH amounts after ingestion of yeast containing S-adenosylmethionine)/(mean value of urinary GH amounts after ingestion of placebo)×100

A mean value of the change rates of the urinary GH amounts in the 13 panelists was 116.2%.

Example 2 and Comparative Example 2

The test and the evaluation were carried out in the same manner as in Example 1, except that samples having the composition shown in Table 2 were used.

Table 2

| Ingredients (one dosage) * | Example 2 | Comparative Example 2 |
|---|---|---|
| Content of S-adenosylmethionine (mg) in yeast containing S-adenosylmethionine | 40.0 | 0.0 |
| Weight (mg) of yeast containing S-adenosylmethionine (dry yeast) | 765.0 | 0.0 |
| Excipient (crystalline cellulose) (mg) | 1035.0 | 1800.0 |
| *Amount in 9 tablets | | |

A change rate (%) of the urinary GH amounts was calculated for each panelist using the formula (1). A mean value of the change rates (%) of the urinary GH amounts in 13 panelists was 112.4%.

The results in Examples 1 and 2 and Comparative Examples 1 and 2 indicate that the GH secretion in vivo is effectively promoted by administering S-adenosylmethionine and/or the salt thereof to the living body.

Formulation examples of various compositions (final products) using the growth hormone secretagogue of the present invention will be shown below. In the following formulation examples, "SAMe-rich dry yeast (trade name)" manufactured by Mitsubishi Gas Chemical Company Inc. was used as the yeast containing S-adenosylmethionine.

(Formulation example 1: Tablet (1))

Tablets were produced, according to a standard method, by tableting 100 mg of the yeast containing S-adenosylmethionine, 60 mg of lactose, 80 mg of crystalline cellulose, 5 mg of carboxymethylcellulose Ca, and 5 mg of sucrose fatty acid ester.

(Formulation example 2: Tablet (2))

Tablets were produced by the standard method using the following materials: 108 mg of granulated material, 30 mg of the yeast containing S-adenosylmethionine, 110 mg of sorbitol, 15 mg of partly pregelatinized starch, 75 mg of magnesium phosphate, 3 mg of calcium stearate, 40 mg of menthol particles, and 5 mg of aspartame.
The granulated material was produced by adding 4000 g of an aqueous solution of 6% by mass of hydroxypropylcellulose to 1935 g of erythritol and 300 g of corn starch. An average particle diameter was 290 μm.

(Formulation example 3: Soft capsule (1))

A content in a soft capsule was produced using the following materials: 200 mg of the yeast containing S-adenosylmethionine, 110 mg of plant fats and oils, 10 mg of glycerin fatty acid ester, and 10 mg of beeswax.
The soft capsule was produced by a standard method using the above content and porcine gelatin.

Examples 3 and 4, and Comparative Example 3

A tablet combining a grape extract was prepared as a sample of Example 3. A capsule agent combining a melinjo extract was prepared as a sample of Example 4. A placebo tablet containing neither the grape extract nor the melinjo extract was prepared as a sample of Comparative Example 3. "VINEATROL20M (trade name)" manufactured by ACTICHEM Co. was used as the grape extract, and "Melinjo Resveratrol-20 (trade name)" manufactured by Luna YBF Corporation Inc. was used as the melinjo extract.
Details of each composition of the samples of the growth hormone secretagogue according to the present invention in Examples and Comparative Example are as shown in following Table 3.

Table 3

| Ingredient (one dosage) (amount in one capsule) | Example 3 | Example 4 | Comparative Example 3 |
|---|---|---|---|
| Grape extract (content of stilbene-based compound) (mg) | 70 (14) | 0 | 0 |
| Melinjo extract extracted from (content of stilbene-based compound) (mg) | 0 | 70 (14) | 0 |
| Excipient (crystalline cellulose) (mg) | 180 | 180 | 250 |

(Evaluation of effect of promoting growth hormone secretion)

Six adult males and females as panelists ingested the sample of Example 3 one hour before going to bed for consecutive 4 days, and the amount of the growth hormone in the first urine (early morning first urine) upon wake-up in the following morning was measured. The same six male and female panelists ingested the sample of Comparative Example 3 one hour before going to bed for consecutive 4 days in the week subsequent to the ingestion of the sample of Example 3, and the amount of the growth hormone in the first urine (early morning first urine) upon wake-up in the following morning was measured.
Further after a while, the same six male and female panelists ingested the sample of Example 4 one hour before going to bed for consecutive 4 days, and the amount of the growth hormone in the first urine (early morning first urine) upon wake-up in the following morning was measured. The same six male and female panelists ingested the sample of Comparative Example 3 one hour before going to bed for consecutive 4 days in the week subsequent to the ingestion of the sample of Example 4, and the amount of the growth hormone in the first urine (early morning first urine) upon wake-up in the following morning was measured.
The administration of each sample and the measurement were repeated 4 times per panelist. The average sleeping time of each panelist was 6.5 hours, a difference between the sleeping time of the panelist whose sleeping time was the longest and the sleeping time of the panelist whose sleeping time was the shortest was 2 hours, and the sleeping time of each panelist was not much different.
The effect of promoting the growth hormone secretion was evaluated by the increase of the amount of the growth hormone (GH) in the urine.

(Measurement of GH amount in urine)

The GH amount in the urine was measured using the chemiluminescence enzyme immunoassay (CLEIA

method) in the same manner as in Example 1. The measurement was contracted out to the company for clinical laboratory testing (SRL Inc.).

A mean value (mean value of urinary GH amounts after ingestion of the sample of Example 3 or the sample of Example 4, respectively) of measured values (4 times) of the urinary GH amount (pg/mg·Cr) was calculated for each panelist. In Comparative Example 3, a mean value (mean value of urinary GH amounts after ingestion of placebo) was also similarly measured. A change rate (%) of the urinary GH amounts was calculated for each panelist using a following formula (2).

Formula (2):

Change rate (%) of urinary GH amounts = (mean value of urinary GH mounts after ingestion of the grape extract or the melinjo extract)/(mean value of urinary GH amounts after ingestion of placebo)×100

The mean values of the change rates of the urinary GH amounts after the ingestion of the grape extract and the melinjo extract in the six panelists were 165.1% and 109.4%, respectively.

The results in Examples 3 and 4 and Comparative Example 3 indicate that the GH secretion in *vivo* is effectively promoted by administering the stilbene-based compound to the living body.

Formulation examples of various compositions (final products) using the growth hormone secretagogue of the present invention will be shown below. In the following formulation examples, "VINEATROL20M (trade name)" manufactured by ACTICHEM Co. was used as the grape extract, and "Melinjo Resveratrol-20 (trade name)" manufactured by Luna YBF Corporation Inc. was used as the melinjo extract.

(Formulation example 4: Tablet (3))

Tablets were produced, according to the standard method, by tableting 140 mg of the grape extract, 155 mg of crystalline cellulose, and 5 mg of sucrose fatty acid ester.

(Formulation example 5: Soft capsule (2))

A content in a soft capsule was produced using the following materials: 140 mg of the grape extract, 110 mg of plant fats and oils, 10 mg of glycerin fatty acid ester, and 10 mg of beeswax.

The soft capsule was produced by the standard method using the above content and porcine gelatin.

(Formulation example 6: Tablet (4))

Tablets were produced, according to the standard method, by tableting 140 mg of the melinjo extract, 155 mg of crystalline cellulose, and 5 mg of sucrose fatty acid ester.

(Formulation example 7: Soft capsule (3))

A content in a soft capsule was produced using the following materials: 140 mg of the melinjo extract, 110 mg of plant fats and oils, 10 mg of glycerin fatty acid ester, and 10 mg of beeswax.

The soft capsule was produced by the standard method using the above content and porcine gelatin.

Example 5 and Comparative Example 4

A capsule agent combining an olive extract was prepared as a sample of Example 5. A placebo capsule agent containing no olive extract was prepared as a sample of Comparative Example 4. "Olivex (trade name)" manufactured by GROUPE GRAP'SUD (France) was used as the olive extract.

Details of each composition of the samples of the growth hormone secretagogue according to the present invention in Example and Comparative Example are as shown in following Table 4.

Table 4

| Ingredients (one dosage)* | Example 5 | Comparative Example 4 |
|---|---|---|
| Olive extract (mg) (Content of polyphenol) (mg) [Content of hydroxytyrosol, tyrosol, and oleuropein](mg) | 100 (30) [10] | 0 |
| Excipient (crystalline cellulose) (mg) | 150 | 250 |
| *Amount in one capsule | | |

(Evaluation of effect of promoting growth hormone secretion)

Six adult males and females as panelists ingested the sample of Example 5 one hour before going to bed, and the amount of the growth hormone in the first urine (early morning first urine) upon wake-up in the following morning was measured. The same 6 panelists as in Example 5 ingested the sample of Comparative Example 4 one hour before going to bed, and the amount of the growth hormone in the first urine upon wake-up in the following morning was measured. The administration of each sample and the measurement were repeated four times per panelist. The average sleeping time of each panelist was 6.5 hours, a difference between the sleeping time of the panelist whose sleeping time was the longest and the sleeping time of the panelist whose sleeping time was the shortest was 2 hours, and the sleeping time of each panelist was not much different.

The effect of promoting the growth hormone secretion was evaluated by the increase of the amount of the growth hormone (GH) in the above urine.

(Measurement of GH amount in urine)

The GH amount in the urine was measured using the chemiluminescence enzyme immunoassay (CLEIA method) in the same manner as in Example 1. The measurement was contracted out to the company for clinical laboratory testing (SRL Inc.).

A mean value (mean value of urinary GH amounts after ingestion of the sample of Example 5) of measured values (4 times) of the urinary GH amount (pg/mg·Cr) was calculated for each panelist. In Comparative Example 4, a mean value (mean value of urinary GH amounts after ingestion of placebo) was also measured. A change rate (%) of the urinary GH amounts was calculated for each panelist using a following formula (3).

Formula (3):

Change rate (%) of urinary GH amounts = (mean value of urinary GH amounts after ingestion of olive extract)/(mean value of urinary GH amounts after ingestion of placebo)×100

The mean value of the change rates of the urinary GH amounts after the ingestion of the olive extract in the six panelists was 254.7%.

The results of Example 5 and Comparative Example 4 indicate that the GH secretion *in vivo* is effectively promoted by administering the olive extract to the living body.

Formulation examples of various compositions (final products) using the growth hormone secretagogue of the present invention will be shown below. In the following formulation examples, "Olivex (trade name)" manufactured by GROUPE GRAP'SUD (France) was used as the olive extract.

(Formulation example 8: Tablet (5))

Tablets were produced, according to the standard method, by tableting 100 mg of the olive extract, 190 mg of crystalline cellulose, 5 mg of carboxymethylcellulose Ca, and 5 mg of sucrose fatty acid ester.

(Formulation example 9: Tablet (6))

Tablets were produced by the standard method using the following materials: 108 mg of granulated material,

100 mg of the olive extract, 110 mg of sorbitol, 15 mg of partly pregelatinized starch, 75 mg of magnesium phosphate, 3 mg of calcium stearate, 40 mg of menthol particles, and 5 mg of aspartame.

The granulated material was produced by adding 4000 g of an aqueous solution of 6% by mass of hydroxypropylcellulose to 1935 g of erythritol and 300 g of corn starch. The average particle diameter was 290 μm.

(Formulation example 10: Soft capsule (4))

A content in a soft capsule was produced using the following materials: 100 mg of the olive extract, 110 mg of plant fats and oils, 10 mg of glycerin fatty acid ester, and 10 mg of beeswax.

The soft capsule was produced by the standard method using the above content and porcine gelatin.

Example 6 and Comparative Example 5

Tablets combining a Siberian larch extract (60 mg of the Siberian larch extract was contained in 6 tablets) were prepared as samples of Example 6. A placebo tablet containing no Siberian larch extract was prepared as a sample of Comparative Example 5. "Jikuberuchin (trade name)" manufactured by Furabiru's was used as the Siberian larch extract. The Siberian larch extract included dihydroquercetin, dihydrokaempferol and naringenin in amounts of 90% by mass, 8% by mass and 2% by mass, respectively.

Details of each composition of the samples of the growth hormone secretagogue according to the present invention in Example and Comparative Example are as shown in following Table 5.

Table 5

| Ingredients (one dosage)* | Example 6 | Comparative Example 5 |
|---|---|---|
| Content of Siberian larch extract (mg) | 60 | 0 |
| Excipient (crystalline cellulose) (mg) | 1440 | 1500 |
| *Amount in 6 tablets | | |

(Evaluation of effect of promoting growth hormone secretion)

Thirteen adult males and females as panelists ingested the sample of Example 6 one hour before going to bed, and the amount of the growth hormone in the first urine (early morning first urine) upon wake-up in the following morning was measured. The same 13 panelists as in Example 6 ingested the sample of Comparative Example 5 one hour before going to bed, and the amount of the growth hormone in the first urine upon wake-up in the following morning was measured. The administration of each sample and the measurement were repeated three times per panelist. The average sleeping time of each panelist was 6.5 hours, the difference between the sleeping time of the panelist whose sleeping time was the longest sleeping time and the sleeping time of the panelist whose sleeping time was the shortest sleeping time was 2 hours, and the sleeping time of each panelist was not much different.

The effect of promoting the growth hormone secretion was evaluated by the increase of the amount of the growth hormone (GH) in the urine.

(Measurement of GH amount in urine)

The GH amount in the urine was measured using the chemiluminescence enzyme immunoassay (CLEIA method) in the same manner as in Example 1. The measurement was contracted out to the company for clinical laboratory testing (SRL Inc.).

A mean value (mean value of urinary GH amounts after ingestion of the sample of Example 6) of measured values (3 times) of the urinary GH amount (pg/mg·Cr) was calculated for each panelist. In Comparative Example 5, a mean value (mean value of urinary GH amounts after ingestion of placebo) was also similarly measured. A change rate (%) of the urinary GH amounts was calculated for each panelist using a following formula (4).

Formula (4):

$$\text{Change rate (\%) of urinary GH amounts for each panelist} = (\text{mean value of urinary GH amounts after ingestion of Siberian larch extract})/(\text{mean value of urinary GH amounts after ingestion of placebo})\times 100$$

The mean value of the change rates of the urinary GH amounts after the ingestion of the Siberian larch extract in the 13 panelists was 123.1%.

The results in Example 6 and Comparative Example 5 indicate that the GH secretion *in vivo* is effectively promoted by administering the Siberian larch extract to the living body.

Formulation examples of various compositions (final products) using the growth hormone secretagogue of the present invention will be shown below. In the following formulation examples, "Jikuberuchin (trade name)" manufactured by Furabiru's was used as the Siberian larch extract.

(Formulation example 11: Tablet (7))

Tablets were produced, according to the standard method, by tableting 10 mg of the Siberian larch extract, 60 mg of lactose, 170 mg of crystalline cellulose, 5 mg of carboxymethylcellulose Ca, and 5 mg of sucrose fatty acid ester.

(Formulation example 12: Tablet (8))

Tablets were produced by the standard method using the following materials: 108 mg of granulated material, 20 mg of the Siberian larch extract, 110 mg of sorbitol, 15 mg of partly pregelatinized starch, 75 mg of magnesium phosphate, 3 mg of calcium stearate, 40 mg of menthol particles, and 5 mg of aspartame.

The granulated material was produced by adding 4000 g of an aqueous solution of 6% by mass of hydroxypropylcellulose to 1935 g of erythritol and 300 g of corn starch. The average particle diameter was 290 $\mu$m.

(Formulation example 13: Soft capsule (5))

A content of a soft capsule was produced using the following materials: 20 mg of the Siberian larch extract, 110 mg of plant fats and oils, 10 mg of glycerin fatty acid ester, and 10 mg of beeswax.

The soft capsule was produced by the standard method using the above content and porcine gelatin.

**INDUSTRIAL APPLICABILITY**

According to the present invention, it is possible to provide the growth hormone secretagogue that is orally available and excellent in effect of promoting the growth hormone secretion.

**Claims**

1. A growth hormone secretagogue comprising one or more selected from the group consisting of S-adenosylmethionine and/or a salt thereof, a Siberian larch extract, an olive extract, and a stilbene-based compound as an active ingredient.

2. The growth hormone secretagogue according to claim 1, wherein S-adenosylmethionine and/or the salt thereof is chemically synthesized S-adenosylmethionine and/or salt thereof.

3. The growth hormone secretagogue according to claim 1, wherein S-adenosylmethionine and/or the salt thereof is a microbial cell containing S-adenosylmethionine and/or a salt thereof, or a culture thereof.

4. The growth hormone secretagogue according to claim 3, wherein the microbial cell is yeast.

5. The growth hormone secretagogue according to claim 1, wherein the stilbene-based compound is an extract extracted from a plant containing the stilbene-based compound.

6. The growth hormone secretagogue according to claim 5, wherein the extract extracted from the plant containing the stilbene-based compound is an extract extracted from a *Vitaceae* plant containing the stilbene-based compound or an extract extracted from a *Gnetaceae* plant containing the stilbene-based compound.

7. The growth hormone secretagogue according to claim 1, wherein the olive extract is an extract separated from a fruit of olive.

8. The growth hormone secretagogue according to claim 1, wherein the olive extract is an olive extract comprising one or more polyphenols selected from the group consisting of hydroxytyrosol, tyrosol, and oleuropein.

9. A food and drink product, a pharmaceutical product or a quasi-pharmaceutical product comprising one or more selected from the group consisting of S-adenosylmethionine and/or a salt thereof, a Siberian larch extract, an olive extract, and a stilbene-based compound.

10. A method for promoting secretion of a growth hormone *in vivo* by administering one or more selected from the group consisting of S-adenosylmethionine and/or a salt thereof, a Siberian larch extract, an olive extract, and a stilbene-based compound to a living body.

**INTERNATIONAL SEARCH REPORT**

International application No.
PCT/JP2012/055361

A. CLASSIFICATION OF SUBJECT MATTER
*A61K31/7076*(2006.01)i, *A61K36/06*(2006.01)i, *A61P5/10*(2006.01)i, *A61P43/00*(2006.01)i

According to International Patent Classification (IPC) or to both national classification and IPC

B. FIELDS SEARCHED

Minimum documentation searched (classification system followed by classification symbols)
A61K31/7076, A61K36/06, A61P5/10, A61P43/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2012 |
| Kokai Jitsuyo Shinan Koho | 1971-2012 | Toroku Jitsuyo Shinan Koho | 1994-2012 |

Electronic data base consulted during the international search (name of data base and, where practicable, search terms used)
CA/MEDLINE/EMBASE/BIOSIS(STN), JSTPlus/JMEDPlus/JST7580(JDreamII)

C. DOCUMENTS CONSIDERED TO BE RELEVANT

| Category* | Citation of document, with indication, where appropriate, of the relevant passages | Relevant to claim No. |
|---|---|---|
| X | D'ANIELLO,A. et al, Occurrence of D-aspartic acid and N-methyl-D-aspartic acid in rat neuroendocrine tissues and their role in the modulation of luteinizing hormone and growth hormone release, FASEB J, 2000, Vol.14, No.5, p.699-714, See RESULTS, DISCUSSION | 1-4,9 |
| X | JP 2004-171 A (Towa Shoji Kabushiki Kaisha), 08 January 2004 (08.01.2004), claims; paragraphs [0007], [0024]; examples (Family: none) | 1,5,9 |
| X | CN 101322809 A (CHI Z.), 17 December 2008 (17.12.2008), claims (Family: none) | 1,6,9 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 25 April, 2012 (25.04.12) | 22 May, 2012 (22.05.12) |

| Name and mailing address of the ISA/ | Authorized officer |
|---|---|
| Japanese Patent Office | |
| Facsimile No. | Telephone No. |

Form PCT/ISA/210 (second sheet) (July 2009)

INTERNATIONAL SEARCH REPORT

International application No. PCT/JP2012/055361

C (Continuation). DOCUMENTS CONSIDERED TO BE RELEVANT

| Category* | Citation of document, with indication, where appropriate, of the relevant passages | Relevant to claim No. |
|---|---|---|
| X<br>A | Cacciari E.,et al., Studio dell'asse entero-insulare nel bambino normale e obeso, Minerva Pediatrica, 1971, 23(24), 1053-60, See TABELLA 2, Fig.5 | 1,7,9<br>8 |
| X | JP 2008-11803 A (Fujifilm Corp.), 24 January 2008 (24.01.2008), examples (Family: none) | 9 |
| X | JP 2008-92869 A (Toyo Sugar Co., Ltd.), 24 April 2008 (24.04.2008), paragraph [0006]; examples (Family: none) | 9 |
| X | JP 2009-227616 A (Gakko Hojin Toin Gakuen), 08 October 2009 (08.10.2009), claims; examples (Family: none) | 9 |
| X | JP 2008-255040 A (Shiseido Co., Ltd.), 23 October 2008 (23.10.2008), examples (Family: none) | 9 |
| X<br>A | JP 2010-500965 A (DSM IP Assets B.V.), 14 January 2010 (14.01.2010), claims; paragraphs [0071] to [0075]; examples<br>& WO 2008/006589 A2 & EP 2049134 A2<br>& KR 2009033470 A & IN 200900790 P1<br>& CN 101516386 A & US 2010055218 A1 | 9<br>8 |
| X<br>Y | Hossdorf T., et al., Sekretionsstimulation von Vasoaktivem Intestinalem Polypeptid (VIP), Verhandlungen der Deutschen Gesellschaft fuer Innere Medizin, 1979, 85, 167-74, See Abb.3 | 9<br>1,7 |
| Y | CHIHARA,K. et al, Effect of vasoactive intestinal polypeptide on growth hormone secretion in perifused acromegalic pituitary adenoma tissues, J Clin Endocrinol Metab, 1982, Vol.54, No.4, p.773-9, See ABSTRACT, Results | 1,7 |

Form PCT/ISA/210 (continuation of second sheet) (July 2009)

**INTERNATIONAL SEARCH REPORT**

International application No.
PCT/JP2012/055361

**Box No. II Observations where certain claims were found unsearchable (Continuation of item 2 of first sheet)**

This international search report has not been established in respect of certain claims under Article 17(2)(a) for the following reasons:

1. [X] Claims Nos.: 10
because they relate to subject matter not required to be searched by this Authority, namely:
Claim 10 pertains to "method for treatment of the human body by surgical operation or therapy" and thus relates to a subject matter on which it is not required to carry out an international search under the provision of PCT Rule 39.1(iv).

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:
The inventions of claims 1-7 and 9 cannot be considered to be novel as disclosed in the following documents, and therefore have no special technical feature.
Note:
FASEB J, 2000, Vol.14, No.5, p.699-714, JP 2004-171 A, CN 101322809 A, Minerva Pediatrica, 1971, 23(24), 1053-60, JP 2008-11803 A, JP 2008-92869 A, JP 2009-227616 A, JP 2008-255040 A, JP 2010-500965 A, Verhandlungen der Deutschen Gesellschaft fuer Innere Medizin, 1979, 85, 167-74

1. [ ] As all required additional search fees were timely paid by the applicant, this international search report covers all searchable claims.
2. [X] As all searchable claims could be searched without effort justifying additional fees, this Authority did not invite payment of additional fees.
3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest**
[ ] The additional search fees were accompanied by the applicant's protest and, where applicable, the payment of a protest fee.
[ ] The additional search fees were accompanied by the applicant's protest but the applicable protest fee was not paid within the time limit specified in the invitation.
[ ] No protest accompanied the payment of additional search fees.

Form PCT/ISA/210 (continuation of first sheet (2)) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2007131541 A
- JP 2006347946 A
- JP 2004269361 A
- RU 2091076


### Non-patent literature cited in the description


- *Lancet,* 1992, vol. 338, 1523-1526
- *Br. J. cancer,* 2002, vol. 86, 774-778
- *Am. J. Epidemiol,* 2008, vol. 167, 648-691
- *Eur. J. Pharmacol.,* 2011, vol. 667, 258-264
- *Nature,* 2006, vol. 444, 337-342
- *Free Radical Biology & Medicine,* 2006, vol. 40, 608-616
- *J. Nutr.,* 2005, 1475-1479
- Clinical significance of measurement of growth hormone in urine. **YASUKO ITAGANE et al.** Pediatrics of Japan. Kanehara & Co., Ltd, 1989, vol. 80, 629-636
- **ADACHI et al.** Clinical Endocrinolog. 1997, vol. 45, 223